# DEMANDE DE BREVET EUROPEEN

(11) **EP 1 568 675 A1**
(43) Date de publication de la demande: **31.08.2005**
(21) Numéro de dépôt: 05290190.7
(22) Date de dépôt: 27.01.2005
(51) Int. Cl.: C07C 2/66, C07C 15/107

(54) **Procédé de production de phénylalcanes utilisant une coupe hydrocarbonee issue du procédé Fischer-Tropsch**

(30) Priorité: 19.02.2004 FR 0401746
(71) Demandeur: Institut Français du Pétrole, 92852 Rueil Malmaison Cédex (FR)
(72) Inventeur: Briot Patrick, Beaurepaire (FR); Guillon Emmanuelle, 69390 Vernaison (FR); Hughes Francois, Charly 69390 Vernaison (FR); Marion Marie-Claire, 69390 Vernaison (FR)

(57) **Abrégé**

On décrit un procédé de production de phénylalcanes comprenant une réaction d'alkylation d'au moins un composé aromatique par au moins une coupe hydrocarbonée directement issue du procédé Fischer-Tropsch comprenant des oléfines linéaires ayant de 9 à 16 atomes de carbone par molécule et des composés oxygénés. Ladite réaction d'alkylation est réalisée dans un réacteur catalytique contenant au moins une zone réactionnelle comportant au moins un catalyseur solide acide et ladite coupe hydrocarbonée ne subit aucun traitement de purification préalablement à son introduction dans ladite zone réactionnelle.

## Description

### Domaine de l'invention

La présente invention concerne un procédé de production de phénylalcanes par alkylation d'au moins un composé aromatique, de préférence du benzène, au moyen d'une coupe d'hydrocarbures directement issue du procédé Fischer-Tropsch et comprenant en général de 9 à 16 atomes de carbone par molécule et préférentiellement de 10 à 14 atomes de carbone par molécule.

Selon un exemple d'application non limitatif, les phénylalcanes obtenus selon le procédé de l'invention pourront constituer des précurseurs pour la formulation de détergents et en particulier de certains détergents biodégradables, par exemple après sulfonation.

Actuellement, les bases pour détergents biodégradables font largement appel aux alkylbenzènes linéaires. La production de ce type de composés est en croissance régulière. Une des propriétés principales recherchée pour ces composés, après une étape de sulfonation, est, outre leur pouvoir détergent, leur biodégradabilité. Pour assurer une biodégradabilité maximale, le groupement alkyle doit être linéaire et long, et la distance entre le groupe sulfonate et le carbone terminal de la chaîne linéaire doit être maximale. Les agents d'alkylation du benzène les plus intéressants sont constitués par les oléfines linéaires en C₉-C₁₆, et de préférence en C₁₀-C₁₄.

Les alkylbenzènes linéaires (LAB), généralement obtenus par alkylation du benzène au moyen d'oléfine(s) linéaire(s), sont préparés le plus souvent selon l'un des deux procédés bien connus de l'homme du métier. Les LAB sont des mélanges d'isomères de phénylalcanes.
Le premier procédé, décrit par exemple dans l'encyclopédie Ullmann 5 ^{ième} volume A 25 page 766 utilise, lors de l'étape d'alkylation du benzène, de l'acide fluorhydrique comme catalyseur acide.
Le second procédé, décrit par exemple dans l'encyclopédie Ullmann 5 ^{ième} volume A 25 page 766 utilise un catalyseur de type Friedel et Craft, généralement à base de AlCl₃.
Ces deux types de catalyseur étaient mis en oeuvre à l'état de solution dans le milieu réactionnel.
Ces deux procédés conduisent à la formation d' isomères de phénylalcanes, par exemple des isomères 2-, 3-, 4-, 5-, 6- phénylalcanes.
Le principal inconvénient de ces procédés est lié à des contraintes d'environnement.

Le premier procédé, basé sur l'utilisation d'acide fluorhydrique pose des problèmes de sécurité d'une part et de retraitement de déchets d'autre part.
Le second procédé pose le problème des rejets issus de l'utilisation desdits catalyseurs de type Friedel et Craft. En effet, il est nécessaire dans ce cas de neutraliser les effluents par une solution basique en sortie de réacteur. De plus, la séparation du catalyseur des produits de la réaction est nécessaire et difficile à mettre en oeuvre pour les deux procédés.

### Etat de la technique

Pour résoudre ces inconvénients, il a été proposé une alkylation du benzène par les oléfines linéaires en présence d'un catalyseur solide.
L'art antérieur fait par exemple état de l'utilisation de catalyseurs possédant des propriétés de sélectivité géométrique et conduisant à une sélectivité améliorée en 2- et 3-phénylalcanes. Les catalyseurs présentant des propriétés de sélectivité géométrique sont généralement constitués de composés zéolithiques tels que définis dans la classification "Atlas of Zeolite Structure Types", W. M Meier, D. H. Olson and Ch. Baerlocher, 5th revised edition, 2001, Elsevier auquel se réfère également la présente demande. Ainsi le brevet US 4,301,317 propose une série de zéolithes parmi lesquels la cancrinite, la gmélinite, la mordénite, l'offrétite et la ZSM-12.
La demande de brevet FR-A-2 697 246 propose d'utiliser des catalyseurs à base de zéolithe Y désaluminée.
La demande de brevet EP-A- 160 144 divulgue d'autre part l'utilisation de zéolithes Y dont la cristallinité varie de 30 à 80 %, tandis que le brevet US 5,036,033 enseigne l'utilisation de zéolithes Y riches en cations ammonium.
La production de phénylalcanes se fait par alkylation du benzène avec des oléfines linéaires issues pour la plupart de la déshydrogénation de paraffines linéaires issues d'une coupe kérosène comme cela est décrit dans les brevets US 6,417,420 et US 6,479,720. Ces coupes contiennent en général entre 8 et 12% poids d'oléfines diluées dans des paraffines (entre 88 et 92% poids). Cette teneur est limitée par l'équilibre thermodynamique de la réaction de déshydrogénation des paraffines concernées.
Le schéma faisant appel à des paraffines linéaires issues d'une coupe kérosène est complexe et coûteux puisqu'il comprend outre la déshydrogénation des paraffines permettant d'obtenir les oléfines, une hydrogénation sélective permettant de réduire en mono-oléfines les dioléfines formées au cours de l'étape de déshydrogénation des paraffines.
On appelle coupe kérosène une coupe issue de la distillation atmosphérique du brut et distillant dans l'intervalle 150°C à 240°C.
Le but de la présente invention est de proposer un procédé de production de phénylalcanes dont la mise en oeuvre est simplifiée.

### Présentation sommaire de l'invention

Il a été trouvé que l'utilisation d'une coupe hydrocarbonée en C9-C16, préférentiellement d'une coupe hydrocarbonée en C10-C14, issue directement du procédé Fischer-Tropsch, comme charge oléfinique pour l'alkylation d'au moins un composé aromatique, de préférence du benzène, conduisait de façon surprenante à une production d'alkylbenzènes linéaires plus élevée que celle obtenue en utilisant une charge oléfinique provenant de la déshydrogénation de paraffines linéaires et que l'utilisation d'une telle charge permettait en outre une mise en oeuvre simplifié du procédé de production d'alkylbenzènes linéaires.
Aussi la présente invention a pour objet un procédé de production de phénylalcanes comprenant une réaction d'alkylation d'au moins un composé aromatique par au moins une coupe hydrocarbonée directement issue du procédé Fischer-Tropsch comprenant des oléfines linéaires ayant de 9 à 16 atomes de carbone par molécule et des composés oxygénés, ladite réaction étant réalisée dans un réacteur catalytique contenant au moins une zone réactionnelle comportant au moins un catalyseur solide acide, ladite coupe hydrocarbonée n'ayant subi aucun traitement de purification préalablement à son introduction dans ladite zone réactionnelle. Ledit catalyseur solide acide comprend de préférence au moins une zéolithe.

### Description détaillée de l'invention

La présente invention a pour objet un procédé de production de phénylalcanes comprenant une réaction d'alkylation d'au moins un composé aromatique par au moins une coupe hydrocarbonée directement issue du procédé Fischer-Tropsch comprenant des oléfines linéaires ayant de 9 à 16 atomes de carbone par molécule et des composés oxygénés, ladite réaction étant réalisée dans un réacteur catalytique contenant au moins une zone réactionnelle comportant au moins un catalyseur solide acide, ladite coupe hydrocarbonée n'ayant subi aucun traitement de purification préalablement à son introduction dans ladite zone réactionnelle.
Conformément au procédé selon l'invention, le composé aromatique est de préférence le benzène ou le toluène, de préférence le benzène. La coupe hydrocarbonée directement issue du procédé Fischer-Tropsch comprend des oléfines linéaires ayant préférentiellement de 10 à 14 atomes (C10-C14) de carbone par molécule.
Le procédé mis au point par la Demanderesse se propose d'utiliser une coupe hydrocarbonée C9-C16 ou préférentiellement une coupe hydrocarbonée C10-C14 issue directement du procédé Fischer-Tropsch. Le procédé Fischer-Tropsch constitue une des étapes permettant de produire des carburants synthétiques à partir de gaz de synthèse (chaîne GTL). La synthèse Fischer-Tropsch, mise en oeuvre dans le procédé Fischer-Tropsch, est une réaction de polymérisation qui, suivant le catalyseur et les conditions opératoires mises en oeuvre, peut conduire à la formation d'hydrocarbures de type paraffiniques, oléfiniques et également de composés oxygénés (notamment des alcools linéaires) dans diverses proportions.
Les produits issus de la synthèse Fischer-Tropsch couvrent une large gamme en terme de nombre de carbone n, incluant le méthane (n=1) jusqu'à des produits à très longues chaînes de carbone (n ≥ 80). Notamment, le procédé Fischer-Tropsch permet de produire des coupes légères C9-C16 majoritairement paraffiniques, mais contenant également des oléfines, principalement des oléfines alpha, dont la concentration est particulièrement bien adaptée pour la production de phénylalcanes, selon la présente invention.
Par exemple, à titre d'illustration, il est possible d'obtenir par le procédé Fischer-Tropsch une coupe C9-C16 contenant environ 10 à 25 %, de préférence 14 à 25%, et de manière très préférée 17 à 23%, poids d'oléfines, dont la proportion d'oléfines linéaires alpha peut-être supérieure à 65%, et une coupe C10-C14 contenant environ 10 à 30 %, de préférence 14 à 28%, et de manière très préférée 17 à 25%, poids d'oléfines, dont la proportion d'oléfines linéaires alpha peut-être supérieure à 63%. Une telle coupe hydrocarbonée peut être utilisée directement pour la mise en oeuvre du procédé selon l'invention.
Cependant ces coupes contiennent aussi des composés oxygénés, en particulier des alcools généralement linéaires, dont la concentration peut être comprise entre 2 et 7 % poids.
Ces composés oxygénés lors de la réaction d'alkylation d'au moins un composé aromatique, de préférence du benzène, vont se déshydrater et générer de l'eau qui est un poison des catalyseurs acides lors des réactions d'alkylation. Cet impact de l'eau est bien décrit par W. Liang et collaborateurs (Zeolites 17, pp 297-303 (1996)).

Pour éviter ce problème, il est nécessaire d'éliminer les composés oxygénés, et en particulier les alcools, notamment les alcools linéaires, contenus dans les coupes issues du procédé Fischer-Tropsch avant d'utiliser lesdites coupes comme charges oléfiniques dans la réaction d'alkylation d'au moins un composé aromatique, de préférence du benzène.
La Demanderesse propose une utilisation directe des coupes hydrocarbonées issues du procédé Fischer-Tropsch, c'est-à-dire n'ayant subi aucun traitement de purification préalablement à leur introduction dans la zone réactionnelle, et en particulier sans séparation préalable des composés oxygénés. La Demanderesse a constaté de façon surprenante que la présence de ces composés oxygénés dans la coupe hydrocarbonée comprenant des oléfines linéaires ayant de 9 à 16 atomes de carbone, de préférence de 10 à 14 atomes de carbone, par molécule ne nuit pas à l'activité du catalyseur solide acide.
De manière surprenante, et grâce à un choix particulier des conditions opératoires, notamment de la température, le procédé d'alkylation, objet de l'invention, permet d'obtenir d'excellents rendements en phénylalcanes et de maintenir un niveau de stabilité du catalyseur supérieur à celui observé dans les procédés utilisant des coupes traditionnelles issues du kérosène.
Selon le procédé, objet de l'invention, la ou les coupe(s) hydrocarbonée(s) issue(s) du procédé Fischer-Tropsch est(sont) mélangée(s) avec le ou les composé(s) aromatique(s) en amont de la zone réactionnelle.
La coupe hydrocarbonée directement issue du procédé Fischer-Tropsch et employée comme charge oléfinique pour l'alkylation du ou des composé(s) aromatique(s), de préférence du benzène, pour la mise en oeuvre du procédé selon l'invention, contient en général des composés oxygénés dans une proportion représentant jusqu'à 35 % poids de ladite coupe. Lesdits composés oxygénés sont des alcools, des aldéhydes, des cétones, des éthers, des esters et/ou des acides. Ils contiennent de préférence le même nombre d'atomes de carbone que les oléfines linéaires présentes dans ladite coupe directement issue du procédé Fischer-Tropsch, c'est-à-dire qu'ils contiennent de 9 à 16 atomes de carbone et préférentiellement de 10 à 14 atomes de carbone par molécule. De préférence, lesdits composés oxygénés sont des alcools ayant de 9 à 16 atomes de carbone par molécule et de manière très préférée ayant de 10 à 14 atomes de carbone par molécule. Il s'agit avantageusement d'alcools linéaires. La coupe directement issue du procédé Fischer-Tropsch et employée comme charge pour l'alkylation du ou des composé(s) aromatique(s), de préférence du benzène, pour la mise en oeuvre du procédé selon l'invention a pour intervalle de distillation de 150 °C à 300°C, et préférentiellement de 170 °C à 270 °C.

Le réacteur utilisé pour la mise en oeuvre du procédé selon l'invention est un réacteur en lit fixe travaillant avec un large excès de composé aromatique, de préférence le benzène, de manière à limiter la formation des dialkylbenzènes (DAB) et à limiter également l'élévation de température en sortie du réacteur, la réaction étant exothermique.
En général, l'étape d'alkylation est suivie d'au moins une étape de séparation des réactifs en excès, et d'au moins une étape de séparation des composés monoalkylés issus de la réaction.
Ces séparations sont effectuées sur une ou plusieurs colonnes à distiller. Par exemple et selon un mode de réalisation préféré du procédé selon l'invention, on fractionne, à la sortie de la zone réactionnelle, dans une colonne à distiller, le produit obtenu de manière à recueillir séparément:
a) une première fraction renfermant le composé aromatique, de préférence le benzène non converti et de l'eau,
b) une deuxième fraction renfermant au moins l'oléfine linéaire C₉-C₁₆ (de préférence C₁₀-C₁₄) non convertie, ainsi que les paraffines éventuellement initialement présentes dans la charge, ainsi que les composés oxygénés initialement présents dans la coupe directement issue du procédé Fischer-Tropsch,
c) une troisième fraction renfermant des isomères de phénylalcanes qui constitue l'ensemble des monophénylalcanes,
d) une quatrième fraction renfermant au moins un poly-alkylbenzène (ou fraction poly-alkylbenzène), celle ci pouvant être au moins en partie, recyclée vers la zone réactionnelle afin d'être transalkylée (selon une réaction de transalkylation), et on recueille un mélange d'isomères de phénylalcanes.

De manière préférée, la deuxième fraction renfermant au moins une oléfine linéaire C₉-C₁₆ (habituellement C₁₀-C₁₄) non convertie peut être, au moins en partie, recyclée vers la zone réactionnelle.

Selon l'invention, le catalyseur d'alkylation solide acide utilisé dans le procédé d'alkylation peut être cristallisé ou amorphe. Il peut s'agir notamment d'un catalyseur constitué exclusivement d'une phase amorphe, de type silice-alumine, alumine silicée, dopée ou non par des halogènes. Il peut s'agir aussi d'un catalyseur constitué d'une phase argile, argile pontée ou modifiée. Il peut s'agir également d'un catalyseur comprenant au moins une zéolithe de structure cristalline, par exemple ayant une structure telle que définie dans la Classification "Atlas of Zeolite Framework Type", (W. M Meier, D. H. Olson and Ch. Baerlocher, 5th revised edition, 2001, Elsevier).
De préférence, le catalyseur solide acide comprend au moins une zéolithe choisie dans le groupe constitué par les zéolithes de type structural FAU, MOR, MTW, OFF, MAZ, BEA et EUO.
Parmi les zéolithes de type structural FAU, la zéolithe Y et la zéolithe Y échangée avec des terres rares (REY) sont préférées. Parmi les zéolithes de type structural MOR, la zéolithe mordénite est préférée. Parmi les zéolithes de type structural MTW, la zéolithe ZSM-12 est préférée. Parmi les zéolithes de type structural OFF, la zéolithe offrétite est préférée. Parmi les zéolithes de type structural MAZ, la zéolithe ZSM-4 est préférée. Parmi les zéolithes de type structural BEA, la zéolithe beta est préférée, et parmi les zéolithes de type structural EUO, la zéolithe EU-1 est préférée.

De manière très préférée, le catalyseur comprend avantageusement au moins une zéolithe Y désaluminée, de rapport atomique Si/Al global supérieur à 4, de préférence compris entre 8 et 70, et encore plus avantageusement compris entre 15 et 50.
La zéolithe Y désaluminée est généralement employée en mélange avec un liant ou une matrice généralement choisi(e) dans le groupe formé par les argiles, les alumines, la silice, la magnésie, la zircone, l'oxyde de titane, l'oxyde de bore et/ou toute combinaison d'au moins deux de ces oxydes comme la silice-alumine ou la silice-magnésie. Toutes les méthodes connues d'agglomération et de mise en forme sont applicables, telles que, par exemple, l'extrusion, le pastillage, la coagulation en gouttes. Le catalyseur contenu dans la zone réactionnelle du réacteur contient généralement de 1 à 100 %, de préférence de 20 à 98 %, de manière très préférée de 40 à 98 % de ladite zéolithe Y désaluminée et de 0 à 99 %, de préférence de 2 à 80 %, et, par exemple, de 2 à 60 % en poids d'un liant ou d'une matrice. Les zéolithes Y désaluminées et leur préparation sont connues. On pourra par exemple se référer au brevet US 4,738,940.

La zéolithe Y, désaluminée ou non, utilisée dans le procédé selon l'invention est préférentiellement au moins partiellement sous forme acide (zéolithe HY) et est caractérisée par différentes spécifications :
- un rapport atomique Si/Al global supérieur à 4, de préférence compris entre 8 et 70, et de manière encore plus préférée compris entre 15 et 50,
- une teneur en sodium inférieure à 0,25 % poids,
- un paramètre cristallin de la maille élémentaire inférieur à 24,55.10⁻¹⁰ m et, de manière préférée, compris entre 24,20.10⁻¹⁰ m et 24,39.10⁻¹⁰ m,
- une surface spécifique déterminée par la méthode B.E.T. supérieure à environ 300 m²/g et de préférence supérieure à environ 450 m²/g,
- une capacité d'adsorption de vapeur d'eau à 25°C, pour une pression partielle de 3,46 mbar (millibar), supérieure à environ 0,5 % et de préférence, supérieure à environ 3 %.

Les zéolithes Y désaluminées sont par exemple synthétisées, généralement à partir d'une zéolithe NaY, par une combinaison appropriée de deux traitements de base : (a) un traitement hydrothermique qui associe température, la température étant préférentiellement comprise entre 450 et 900°C et très préférentiellement entre 550 et 800°C, et pression partielle de vapeur d'eau (40 à 100% de vapeur d'eau), et (b) un traitement acide par, de préférence, un acide minéral fort et concentré (0,01 à 10 N). L'étape (a) n'est cependant qu'éventuelle. Généralement la zéolithe NaY à partir de laquelle on prépare la zéolithe Y utilisée dans le catalyseur présent dans la zone réactionnelle possède un rapport atomique Si/Al global compris entre environ 1,8 et 3,5 ; il conviendra au préalable d'en abaisser la teneur pondérale en sodium à moins de 3 % et, de préférence, à moins de 2,5 %. L'abaissement de la teneur en sodium peut s'effectuer par échanges ioniques de la zéolithe NaY dans des solutions de sel d'ammonium (nitrate, sulfate, oxalate etc..) de concentration en ammonium comprise entre 0,01 et 10 N, à une température comprise entre 10 et 180°C (échange sous pression autogène éventuellement), pendant une durée supérieure à 10 minutes environ. La zéolithe NaY possède en outre généralement une surface spécifique comprise entre environ 750 et 950 m²/g.

Un autre mode préféré de l'invention consiste à utiliser comme catalyseur solide acide dans la zone réactionnelle du procédé selon l'invention un mélange de zéolithes. Il peut s'agir par exemple d'un mélange de zéolithes constitué d'au moins une zéolithe Y telle que décrite précédemment, et d'au moins une zéolithe de type structural MOR, notamment une zéolithe de type mordénite. La préparation des zéolithes de type structural MOR est connu de l'état de la technique (US 4.503.023). S'agissant de la préparation d'un catalyseur comprenant un mélange de zéolithes, le mélange desdites zéolithes, lesquelles se trouvent à l'état de poudre, est réalisé par toutes les techniques de mélange de poudres connues de l'Homme du métier et suivi de la mise en forme. Lorsque le mélange des poudres de zéolithes est achevé, le mélange est mis en forme par toute technique connue de l'Homme du métier. Il peut en particulier être mélangé à une matrice, généralement amorphe, par exemple à une poudre humide de gel d'alumine. La mise en forme peut aussi être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On peut aussi avantageusement utiliser des mélanges d'alumine et de silice, des mélanges d'alumine et de silice-alumine. On préfère utiliser des matrices contenant de l'alumine, sous toutes ses formes connues de l'Homme du métier, et de manière encore plus préférée l'alumine gamma. Le mélange est ensuite mis en forme. Plusieurs techniques peuvent être employées dans ce but et notamment l'extrusion au travers d'une filière, le pastillage et la dragéification. Le mélange de zéolithes peut également être constitué d'un mélange de zéolithes déjà mises en forme comme décrit précédemment.
Le catalyseur utilisé pour la mise en oeuvre du procédé selon l'invention est mis en forme sous la forme de grains de différentes formes et dimensions. Il est utilisé en général sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes ou de disques.
Après l'étape de mise en forme, le produit obtenu est soumis à une étape de séchage à une température comprise entre 100 et 300°C, de préférence entre 120 et 200°C, puis à une étape de calcination à une température comprise entre 350 et 650°C, de préférence entre 450 et 600°C.

Les conditions opératoires appliquées dans la zone réactionnelle sont bien entendues choisies par l'homme de l'art en fonction de la nature du catalyseur. Habituellement, pour les réactions d'alkylation du benzène, la température est comprise entre 80°C et 180°C. Ceci a été largement décrit dans la littérature. (Applied Catalysis A : General 184, pp 231-238 (1999); Applied Catalysis A : General 238, pp 99-107 (2003)). Compte tenu de la spécificité de la charge oléfinique utilisée pour l'alkylation, la réaction d'alkylation est réalisée à une température supérieure à 180 °C, et de façon préférentielle supérieure à 200 °C. La pression totale est comprise entre 1 et 10 MPa, de préférence entre 2 et 6 MPa, avec un débit d'hydrocarbures liquides (vitesse spatiale) d'environ 0,5 à 50 volumes par volume de catalyseur et par heure et un rapport molaire benzène/oléfine compris entre 1 et 40 .

L'invention sera mieux comprise à la lecture des exemples qui suivent.

### Exemple 1 : alkylation du benzène par une coupe issue de la déshydrogénation d'un kérosène (dans les conditions standard ; exemple comparatif)

On utilise un réacteur contenant 50 cm³ de catalyseur constitué de 40% de zéolithe Y désaluminée, avec un rapport Si/Al voisin de 45 sous forme d'extrudés.
Les conditions opératoires pour l'alkylation du benzène et d'une coupe pétrolière issue de la déshydrogénation d'un kérosène (coupe issue de la distillation atmosphérique du brut et ayant un intervalle de distillation compris entre 150°C et 240°C) sont des conditions opératoires standard connues de l'homme du métier :
- température : 135°C
- pression : 4 MPa
- VVH = 1 h⁻¹
- rapport molaire benzène/oléfine : 10/1

On prépare une charge contenant 32% poids de benzène et 68% poids de coupe kérosène issue de la déshydrogénation. La composition de cette coupe est présentée dans le tableau 1 ci-dessous:

**Tableau 1 :**

| Composition de la coupe issue de la déshydrogénation du kérosène. | | |
|---|---|---|
| Atome de carbone | Oléfines (% Pds) | Paraffines (% Pds) |
| C10 | 1,67 | 15,02 |
| C11 | 3,29 | 29,59 |
| C12 | 2,81 | 25,31 |
| C13 | 2,23 | 20,08 |

En sortie de la zone réactionnelle, les produits sont recueillis et on obtient la composition des effluents présentée dans le tableau 2 ci-dessous.

**Tableau 2 :**

| Composition pondérale des effluents de la réaction. | |
|---|---|
| Composés | teneur pondérale (%) |
| Benzène | 28,6 |
| Paraffines | 61,2 |
| LAB C10 | 1,8 |
| LAB C11 | 3,4 |
| LAB C12 | 2,8 |
| LAB C13 | 2,2 |
| Total LAB | 10,2 |

Un LAB en C10 est un alkylbenzène linéaire dont la chaîne alkyle comporte 10 atomes de carbone. Il s'agit d'un mélange de 2-, 3-, 4-, 5-phénylalcanes dont la chaîne alkyle comporte 10 atomes de carbone. Un LAB en C11 est un alkylbenzène linéaire dont la chaîne alkyle comporte 11 atomes de carbone. Il s'agit d'un mélange de 2-, 3-, 4-, 5-, 6-phénylalcanes dont la chaîne alkyle comporte 11 atomes de carbone. Un LAB en C12 est un alkylbenzène linéaire dont la chaîne alkyle comporte 12 atomes de carbone. Il s'agit d'un mélange de 2-, 3-, 4-, 5-, 6-phénylalcanes dont la chaîne alkyle comporte 12 atomes de carbone. Un LAB en C13 est un alkylbenzène linéaire dont la chaîne alkyle comporte 13 atomes de carbone. Il s'agit d'un élange de 2-, 3-, 4-, 5-, 6-, 7-phénylalcanes dont la chaîne alkyle comporte 13 atomes de carbone.

### Exemple 2 : alkylation du benzène par une coupe Fischer-Tropsch selon l'invention

On utilise un réacteur contenant 50 cm³ de catalyseur constitué de 40% de zéolithe Y désaluminée, avec un rapport Si/Al voisin de 45 sous forme d'extrudés.

La coupe utilisée dans cet exemple pour l'alkylation du benzène a été sélectionnée afin d'avoir le même nombre d'atomes de carbone que la coupe issue de la déshydrogénation du kérosène de l'exemple 1, c'est-à-dire une coupe C10-C13 issue du procédé Fischer-Tropsch. La composition de cette coupe est présentée dans le tableau 3.

**Tableau 3 :**

| Composition pondérale de la coupe Fischer-Tropsch | | | |
|---|---|---|---|
| Nombre d'atome de carbone | Paraffines (% poids) | Oléfines (% poids) | Alcools (% poids) |
| C10 | 17,8 | 7,1 | 1,4 |
| C11 | 18,6 | 5,9 | 1,4 |
| C12 | 18,7 | 4,8 | 1,4 |
| C13 | 18,2 | 3,7 | 1 |
| | | | |
| Total | 73,3 | 21,5 | 5,2 |

La coupe issue du procédé Fischer-Tropsch possède une teneur en oléfines supérieure à celle de la coupe issue de la déshydrogénation d'une coupe kérosène.
De la même façon que précédemment, la charge est mélangée à du benzène de manière à ce que le rapport molaire benzène/oléfines à l'entrée de la zone réactionnelle soit voisin de 10 moles/mole.

Cette charge est injectée dans la zone réactionnelle dans les conditions opératoires suivantes :
- température : 230°C
- pression : 4 Mpa
- VVH = 1 h⁻¹
- rapport molaire benzène/oléfine : 10/1

En sortie de la zone réactionnelle, les produits sont recueillis et on obtient la composition des effluents présentée dans le tableau 4.

**Tableau 4 :**

| Composition pondérale des produits de la réaction. | |
|---|---|
| Composés | Teneur pondérale (%) |
| Benzène | 45,5 |
| Paraffines | 35,3 |
| eau | 0,3 |
| LAB C10 | 6,3 |
| LAB C11 | 5,2 |
| LAB C12 | 4,2 |
| LAB C13 | 3,2 |
| | |
| Total LAB | 18,9 |

Un LAB en C10 est un alkylbenzène linéaire dont la chaîne alkyle comporte 10 atomes de carbone. Il s'agit d'un mélange de 2-, 3-, 4-, 5-phénylalcanes dont la chaîne alkyle comporte 10 atomes de carbone. Un LAB en C11 est un alkylbenzène linéaire dont la chaîne alkyle comporte 11 atomes de carbone. Il s'agit d'un mélange de 2-, 3-, 4-, 5-, 6-phénylalcanes dont la chaîne alkyle comporte 11 atomes de carbone. Un LAB en C12 est un alkylbenzène linéaire dont la chaîne alkyle comporte 12 atomes de carbone. Il s'agit d'un mélange de 2-, 3-, 4-, 5-, 6-phénylalcanes dont la chaîne alkyle comporte 12 atomes de carbone. Un LAB en C13 est un alkylbenzène linéaire dont la chaîne alkyle comporte 13 atomes de carbone. Il s'agit d'un mélange de 2-, 3-, 4-, 5-, 6-, 7-phénylalcanes dont la chaîne alkyle comporte 13 atomes de carbone.

De façon surprenante, il n'a pas été constaté de baisse de l'activité du catalyseur, et de plus la production de phénylalcanes est largement supérieure à celle obtenue lorsque la charge oléfinique utilisée pour l'alkylation est issue de la déshydrogénation d'une coupe kérosène.

## Revendications

1. Procédé de production de phénylalcanes comprenant une réaction d'alkylation d'au moins un composé aromatique par au moins une coupe hydrocarbonée directement issue du procédé Fischer-Tropsch comprenant des oléfines linéaires ayant de 9 à 16 atomes de carbone par molécule et des composés oxygénés, ladite réaction étant réalisée dans un réacteur catalytique contenant au moins une zone réactionnelle comportant au moins un catalyseur solide acide, ladite coupe hydrocarbonée n'ayant subi aucun traitement de purification préalablement à son introduction dans ladite zone réactionnelle.

2. Procédé selon la revendication 1 dans lequel lesdits composés oxygénés sont présents dans une proportion représentant jusqu'à 35% poids de ladite coupe hydrocarbonée directement issue du procédé Fischer-Tropsch.

3. Procédé selon la revendication 1 ou 2 dans lequel lesdits composés oxygénés sont des alcools, des aldéhydes, des cétones, des éthers, des esters et/ou des acides.

4. Procédé selon l'une des revendications 1 à 3 dans lequel lesdits composés oxygénés sont des alcools ayant de 9 à 16 atomes de carbone par molécule.

5. Procédé selon l'une des revendications 1 à 4 dans lequel le composé aromatique est le benzène.

6. Procédé selon l'une des revendications 1 à 5 dans lequel les oléfines linéaires sont des oléfines ayant de 10 à 14 atomes de carbone par molécule.

7. Procédé selon l'une des revendications 1 à 6 dans lequel la réaction d'alkylation est réalisée à une température supérieure à 180°C.

8. Procédé selon la revendication 7 dans lequel la réaction d'alkylation est réalisée à une température supérieure à 200°C.

9. Procédé selon l'une des revendications 1 à 8 dans lequel le catalyseur solide acide comprend au moins une zéolithe choisie dans le groupe constitué par les zéolithes de type structural FAU, MOR, MTW, OFF, MAZ, BEA, EUO.

10. Procédé selon l'une des revendications 1 à 9 dans lequel le catalyseur solide acide comprend au moins une zéolithe Y désaluminée de rapport atomique Si/Al global supérieur à 4.
